# DEMANDE DE BREVET EUROPEEN

(11) **EP 1 661 606 A1**
(43) Date de publication de la demande: **31.05.2006**
(21) Numéro de dépôt: 05292487.5
(22) Date de dépôt: 23.11.2005
(51) Int. Cl.: A63B 69/16, A61B 5/22, G01L 3/24

(54) **Pédale de cycle dynamométrique**

(30) Priorité: 24.11.2004 FR 0412455
(71) Demandeur: Look Cycle International, 58000 Nevers (FR)
(72) Inventeur: Beaulieu, Cyril, F-58660 COULANGES LES NEVERS (FR)
(74) Mandataire: Laget, Jean-Loup

(57) **Abrégé**

La pédale est destinée à la mesure des efforts appliqués lors du pédalage. Elle comporte un corps de pédale (2) pourvu de moyens de fixation (3, 4) d'une chaussure de cycliste et d'un logement (9) recevant un axe de pédale (10) relié à une manivelle de pédalier (11). Plusieurs jauges de déformation (13, 15, 16, 21, 22) sont disposées de part et d'autre de l'axe de pédale (10). La pédale est associée à des moyens de mesure de l'angle de rotation de la manivelle. Le corps de pédale (2) est composé d'une partie supérieure (2A) comportant les moyens de fixation et d'une partie inférieure (2B) comportant le logement. Les parties supérieure et inférieure du corps de pédale sont séparées l'une de l'autre par un interstice (2C) et reliées l'une à l'autre par des organes de liaison verticaux (23) portant des jauges de déformation (15, 16) aptes à mesurer les efforts transversaux.

## Description

La présente invention concerne une pédale de cycle dynamométrique utilisée pour mesurer les efforts appliqués sur la pédale lors du pédalage.

Les pédales dynamométriques permettent d'étudier les différentes variables du pédalage telles que la fréquence, la puissance, les forces appliquées par les membres inférieurs du cycliste, l'efficacité ou le rendement mécanique. L'utilité de ces pédales dynamométriques est qu'elles peuvent, entre autres, contribuer à minimiser les risques de blessure des genoux en permettant de constater les dissymétries qui peuvent exister entre les deux membres inférieurs. Elles permettent également de déterminer la fréquence de pédalage correspondant à une efficacité maximale.

Les pédales dynamométriques peuvent aussi être utilisées pour déterminer un mauvais alignement entre le cycliste et sa bicyclette, encore une fois pour éviter les blessures du genou que l'on peut qualifier de pathologie d'origine mécanique. Elles peuvent en outre être utilisées à des fins thérapeutiques en fournissant un instrument de suivi d'une procédure de correction ou de rééducation.

Les pédales dynamométriques sont également utilisées pour faire une optimisation de la structure de la pédale elle-même, de la manivelle du pédalier qui porte la pédale, et du cadre.

La notion de pédale dynamométrique comprend ici aussi bien les pédales dynamométriques proprement dites que les pédales associées à un dispositif dynamométrique permettant de mesurer les efforts appliqués sur la pédale.

On connaît des pédales dynamométriques permettant l'obtention de certaines données utiles pour le calcul des efforts appliqués sur une pédale. Pratiquement toutes ces pédales sont basées sur une mesure de la déformation de l'axe de pédale à l'aide de jauges de déformation fixées sur celui-ci. Elles ne permettent de mesurer que deux composantes de la force appliquée sur la pédale. Il s'agit de la force perpendiculaire à la surface d'appui de la pédale et de la force longitudinale par rapport à celle-ci. Les déformations n'ayant cependant pas lieu uniquement dans la direction des mesures effectuées par les jauges de déformation, il est ici nécessaire de déterminer en outre une matrice de calibrage afin de calculer les forces en fonction des déformations mesurées.

Un tel dispositif est dans la généralité de son principe connu du DE 101 58 600.

Une pédale dynamométrique est également connue du FR 2 724 728 qui décrit des moyens de mesure des efforts appliqués sur la pédale, ces moyens étant constitués par quatre jauges de déformation fixées sur le pourtour de l'axe de pédale et décalées de 90° d'une jauge à la suivante. Ce dispositif comporte en outre des moyens de détection de la position angulaire de la manivelle de pédalier et propose de déterminer les forces tangentielles s'exerçant sur l'axe de pédale dans deux directions, ainsi que les composantes de la force de pédalage relativement à un référentiel lié à la manivelle de pédalage.

Encore une autre pédale dynamométrique est décrite dans le document DD 294 673 A5 pour la mesure des efforts de pédalage. Elle comporte dans ce but des jauges de contrainte destinées à la mesure d'un composant d'effort de pédalage vertical et d'un seul composant d'effort de pédalage horizontal qui est plus précisément un composant longitudinal. Cependant, cette pédale n'est pas, tout comme les autres pédales connues mentionnées ci-dessus, conçue de manière à permettre l'évaluation de la composante transversale de la force appliquée contre la pédale, alors que cette composante transversale représente une variable importante dans l'analyse biomécanique de pédalage, d'autant plus que les forces transversales produisent un couple de force absolument inutile à la propulsion et constituent ainsi des forces perdues que l'on souhaite minimiser.

Cette composante transversale peut aussi révéler une dissymétrie éventuelle du pédalage, voire même une insuffisance d'un des membres inférieurs ou un problème articulaire. Il apparaît ainsi important de pouvoir tenir compte de cette variable qui est un indice essentiel dans la modélisation et l'analyse de pédalage sur le terrain.

A ce jour, aucune pédale dynamométrique de ce genre ne permet d'effectuer des mesures de toutes les trois composantes des forces appliquées sur les pédales, et en particulier de la composante transversale, alors que cette dernière est indispensable afin de prendre en compte toutes les variables du pédalage des membres inférieurs droit et gauche.

Par ailleurs, toutes les pédales dynamométriques connues sont conçues pour une utilisation en laboratoire et ne permettent pas d'estimer précisément les forces appliquées sur la pédale en conditions réelles sur le terrain.

Le but de l'invention est de proposer une pédale dynamométrique qui remédie aux inconvénients des pédales connues en permettant la mesure complète et exacte de la force appliquée contre la pédale par des moyens extrêmement simples. En plus, la pédale selon l'invention est particulièrement bien adaptée pour être utilisée en condition de fonctionnement normal sur le terrain. Par ailleurs, et pour faciliter cette utilisation sur le terrain, la structure supérieure de la pédale constitue avantageusement toujours une pédale classique dite automatique permettant de fixer automatiquement la chaussure d'un cycliste contre la pédale à l'aide d'une cale de fixation fixée à demeure sous la chaussure et coopérant avec une griffe avant fixe et une griffe arrière précontrainte montée basculante.

L'objet de l'invention est une pédale de cycle dynamométrique pour la mesure des efforts appliqués sur la pédale lors du pédalage, comportant un corps de pédale pourvu de moyens de fixation d'une chaussure de cycliste contre ledit corps de pédale, et d'un logement recevant un axe de pédale apte à être relié à une manivelle de pédalier, plusieurs jauges de déformation étant disposées de part et d'autre de l'axe de pédale et comprennent des jauges de déformation pour mesurer les efforts longitudinaux, ainsi que des jauges pour mesurer la flexion dans un plan vertical pour transformer les efforts reçus en signaux électriques à transmettre à une unité de numérisation des signaux et de mémorisation de données, la pédale étant en outre associée à des moyens de mesure de l'angle de rotation de ladite manivelle de pédalier, le corps de pédale étant composé d'une partie supérieure comportant lesdits moyens de fixation et d'une partie inférieure séparée de ladite partie supérieure et comportant ledit logement, lesdites parties supérieure et inférieure du corps de pédale étant séparées l'une de l'autre par un interstice tout en étant reliées l'une à l'autre par des organes de liaison verticaux, caractérisée par le fait qu'elle comporte en outre des jauges de déformation aptes à mesurer les efforts transversaux et disposées dans ledit interstice en s'étendant dans le sens longitudinal du corps de pédale.

Selon d'autres caractéristiques de l'invention :
- lesdites jauges de déformation aptes à mesurer les efforts transversaux sont portées par lesdits organes de liaison verticaux ;
- ladite partie supérieure du corps de pédale est, sur sa face inférieure, pourvue de pattes verticales plates reliées auxdits organes de liaison et portant des jauges de déformation s'étendant dans le sens transversal du corps de pédale afin de mesurer les efforts longitudinaux ;
- lesdits organes de liaison sont au nombre de trois et deux des organes de liaison sont disposés latéralement à l'avant de ladite partie supérieure du corps de pédale, et le troisième organe de liaison est disposé centralement à l'arrière de ladite partie supérieure ;
- ledit troisième organe de liaison porte en outre une jauge de déformation permettant de mesurer la compression ;
- lesdites pattes sont reliées auxdits organes de liaison par des premières articulations désaccouplant les efforts longitudinaux de ladite partie supérieure du corps de pédale vers le bas ;
- ladite partie inférieure du corps de pédale a la forme d'une fourche dont les branches sont dirigées vers l'avant et portent lesdites jauges de déformation permettant de mesurer la flexion desdites branches dans un plan vertical ;
- chacune desdites branches de ladite fourche comporte dans une partie médiane une fente horizontale s'étendant selon l'axe longitudinal de la branche et limitée à chaque extrémité par une lumière circulaire permettant de déformer la branche selon une ligne brisée ;
- lesdits organes de liaison sont reliés à ladite partie inférieure du corps de pédale à l'aide de deuxièmes articulations désaccouplant les efforts transversaux desdits organes de liaison vers le bas ;
- lesdits organes de liaison sont des plaquettes allongées amincies dans leur partie centrale et s'étendant dans des plans longitudinaux de la pédale ;
- l'extrémité libre desdites pattes a la forme d'une chape apte à recevoir l'extrémité supérieure de l'organe de liaison correspondant, et les branches de ladite chape comportent deux alésages opposés alignés sur un alésage effectué dans l'extrémité supérieure dudit organe de liaison pour recevoir un axe de pivotement transversal s'étendant à travers les trois alésages de manière à former ladite première articulation;
- l'extrémité supérieure de chaque organe de liaison présente une surface incurvée vers le haut ;
- ladite partie inférieure du corps de pédale comporte des évidements dirigés vers le haut et recevant l'extrémité inférieure d'un organe de liaison correspondant, chaque évidement étant pourvu de deux alésages opposés alignés sur un alésage effectué dans ladite extrémité inférieure dudit organe de liaison pour recevoir un axe de pivotement longitudinal s'étendant à travers les trois alésages de manière à former ladite deuxième articulation ;
- ladite unité de numérisation et de mémorisation est intégrée dans le corps de pédale;
- ladite unité de numérisation et de mémorisation comporte un boîtier électronique associé à un support de mémoire amovible ;
- la pédale comporte des moyens de mesure d'angle secondaires aptes à mesurer l'angle de l'axe de pédale par rapport au corps de pédale et à générer des signaux électriques à transmettre à ladite unité de numérisation des signaux et de mémorisation de données ; et
- lesdits moyens de mesure d'angle secondaires sont sous forme d'un codeur angulaire intégré dans le corps de pédale.

D'autres caractéristiques et avantages de l'invention ressortiront de la description qui va suivre d'un mode de réalisation non limitatif de l'invention, en référence aux figures annexées dans lesquelles :
- la figure 1 est une vue éclatée en perspective de dessus montrant les éléments essentiels de la pédale dynamométrique selon l'invention ;
- la figure 2 est une vue partiellement éclatée en perspective de dessus de la pédale de la figure 1 pourvue d'une cale de fixation d'une chaussure de cycliste ;

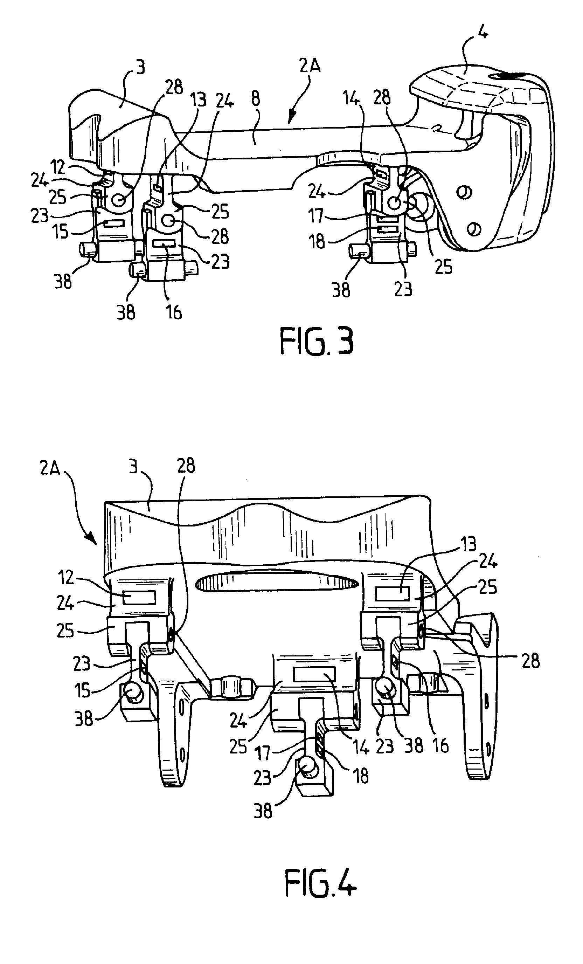

- la figure 3 est une vue en perspective de dessus montrant la partie supérieure de la pédale avec les organes de liaison verticaux aptes à relier la partie supérieure à la partie inférieure ;
- la figure 4 est une vue en perspective de dessous des éléments de la figure 3;

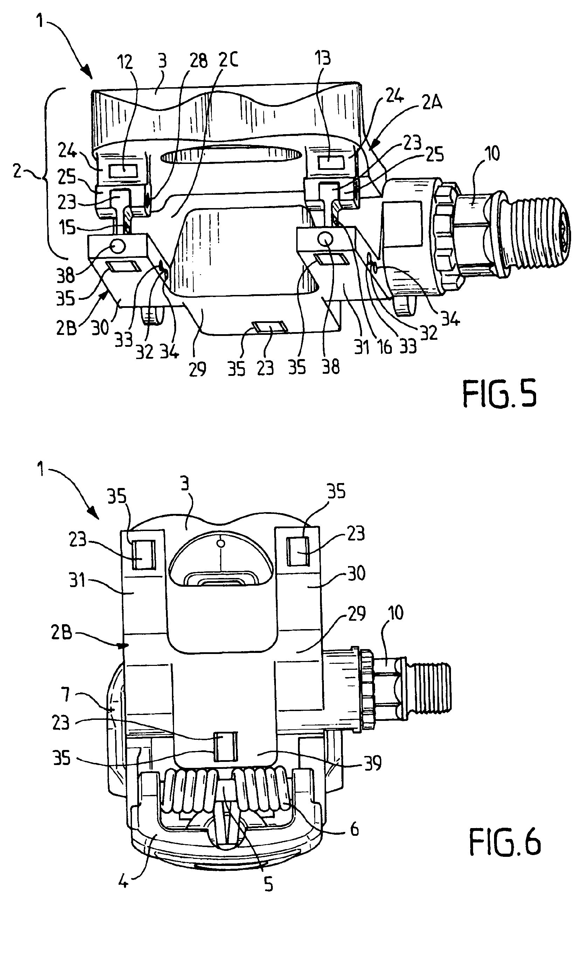

- la figure 5 est une vue en perspective de dessous montrant la partie supérieure et la partie inférieure de la pédale reliées l'une à l'autre par des organes de liaison selon l'invention ;
- la figure 6 est une vue de dessous de la pédale de la figure 5 ;
- la figure 7 est une vue latérale montrant la partie supérieure de la pédale dynamométrique selon l'invention ;
- la figure 8 est une vue de face d'un organe de liaison selon l'invention ;
- la figure 9 est une vue latérale de l'organe de liaison de la figure 8;

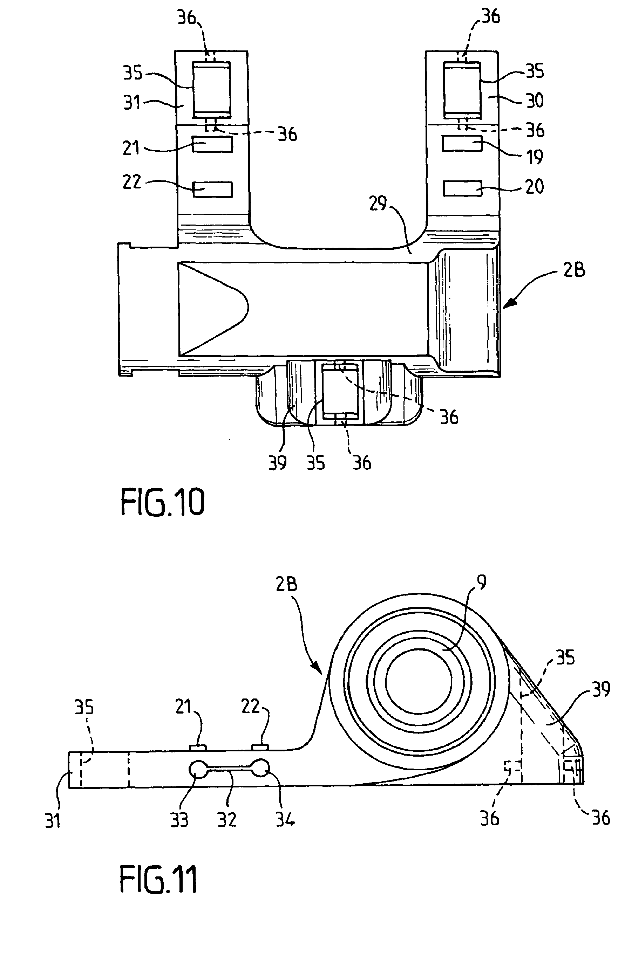

- la figure 10 est une vue de dessus de la partie inférieure de la pédale dynamométrique selon l'invention ; et
- la figure 11 est une vue latérale de la partie inférieure montrée à la figure 12.

Dans les figures, les éléments identiques ou équivalents porteront les mêmes signes de référence.

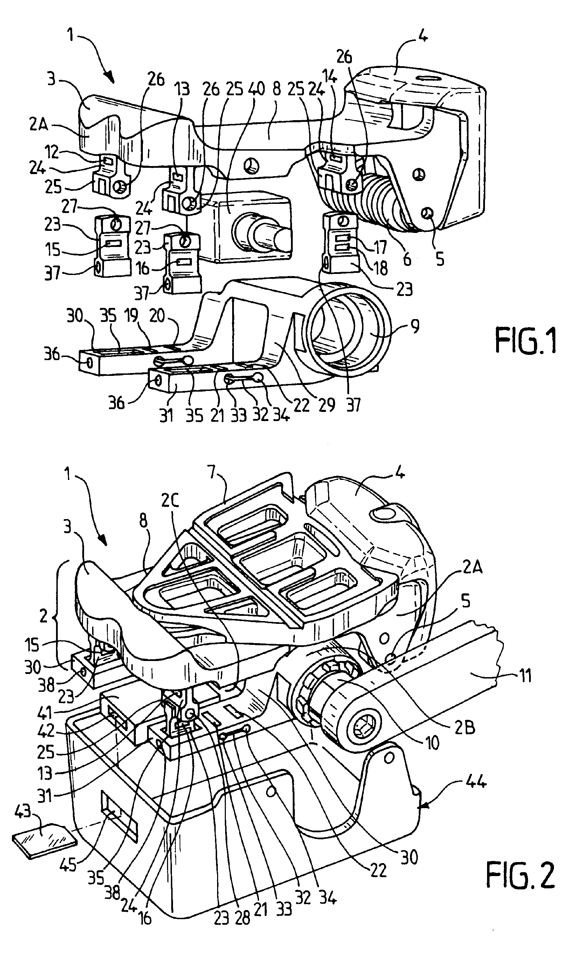

Les figures 1 et 2 montrent les éléments essentiels de la pédale de cycle dynamométrique 1 selon l'invention. La pédale est dans l'exemple illustré une pédale du genre automatique et comporte un corps de pédale 2 pourvu de moyens de fixation d'une chaussure de cycliste (non représentée). Ces moyens de fixation comportent un organe d'enclenchement avant fixe 3 et un organe d'enclenchement arrière 4 monté basculant sur un axe transversal 5 et précontraint par un ressort hélicoïdal 6 enfilé sur cet axe.

Une cale ou plaque de fixation 7 est fixée sous la chaussure de cycliste pour être engagée entre les organes d'enclenchement avant et arrière 3, 4 afin de venir en appui contre une surface d'appui 8 sur la face supérieure du corps de pédale 2 (voir figure 2), alors que la semelle de la chaussure repose sur les organes d'enclenchement avant et arrière.

Le corps de pédale comporte en outre un logement 9 recevant un axe de pédale 10 apte à être relié à une manivelle de pédalier11 montrée partiellement à la figure 2.

Tous ces organes d'une pédale automatique, ainsi que leurs fonctions, sont parfaitement connus dans l'état de la technique et ne seront pas décrits davantage ici.

Plusieurs organes de mesure de contrainte sous forme de jauges de déformation 12 à 22 sont disposés de part et d'autre de l'axe de pédale 10. Les emplacements et les fonctions différentes de ces jauges de déformation seront décrits en détail plus tard.

La pédale est en outre associée à des moyens de mesure de l'angle de rotation de la manivelle de pédalier 11, c'est-à-dire des moyens détectant la position angulaire de la manivelle de pédalier par rapport au cadre de cycle (non représenté). Ces moyens de mesure d'angle ne sont pas représentés sur les figures et ne seront pas décrits puisqu'ils sont bien connus dans la technique. Il peut s'agir de moyens de mesure d'angle tels que décrits dans le FR 2 724 728 déjà mentionné, ou bien des moyens à aimant et détecteur placé sur le cadre respectivement sur la manivelle, alternativement sur la pédale.

Selon une des caractéristiques essentielles de l'invention, le corps de pédale 2 est composé d'une partie supérieure 2A comportant les moyens de fixation 3, 4 de la chaussure de cycliste et d'une partie inférieure 2B séparée de la partie supérieure par un interstice 2C et comportant le logement 9. Ces parties supérieure et inférieure 2A, 2B du corps de pédale sont reliées l'une à l'autre par des organes de liaison verticaux 23. Des jauges de déformation 15, 16, 17 sont disposées dans cet interstice 2C en s'étendant dans le sens longitudinal du corps de pédale afin de mesurer les efforts transversaux.

Dans le mode de réalisation illustré sur les figures, les organes de liaison verticaux 23 portent ces jauges de déformation 15, 16, 17 aptes à mesurer les efforts transversaux.

Ces organes de liaison 23 ont avantageusement la forme de plaquettes allongées s'étendant dans des plans longitudinaux de la pédale. Elles sont amincies dans leur partie centrale où sont disposées les jauges de déformation 15, 16, 17. Les organes de liaison 23 sont au nombre de trois dont deux sont disposés latéralement à l'avant de la partie supérieure 2A du corps de pédale 2 et le troisième organe de liaison est disposé centralement à l'arrière de cette partie supérieure.

Le troisième organe de liaison 23 porte avantageusement en outre une jauge de déformation 18 permettant de mesurer la compression.

Selon une autre caractéristique de l'invention, la partie supérieure 2A du corps de pédale 2 est sur sa face inférieure pourvue de pattes verticales 24 reliées aux organes de liaison 23 et portant des jauges de déformation 12, 13, 14 s'étendant dans le sens transversal du corps de pédale 2 afin de mesurer les efforts longitudinaux. Ces pattes 24 comportent également une partie centrale amincie recevant ces jauges de déformation 12, 13, 14.

Les pattes verticales 24 sont de préférence reliées aux organes de liaison 23 par des premières articulations désaccouplant les efforts longitudinaux de la partie supérieure 2A du corps de pédale 2 vers le bas. Dans ce but, l'extrémité libre des pattes verticales 24 a la forme d'une chape 25 recevant l'extrémité supérieure de l'organe de liaison correspondant 23. Les chapes 25 comportent deux alésages opposés 26 alignés sur un alésage 27 effectué dans l'extrémité supérieure de l'organe de liaison 23 pour recevoir un axe de pivotement transversal 28 s'étendant à travers les trois alésages 26, 27 de manière à former cette articulation.

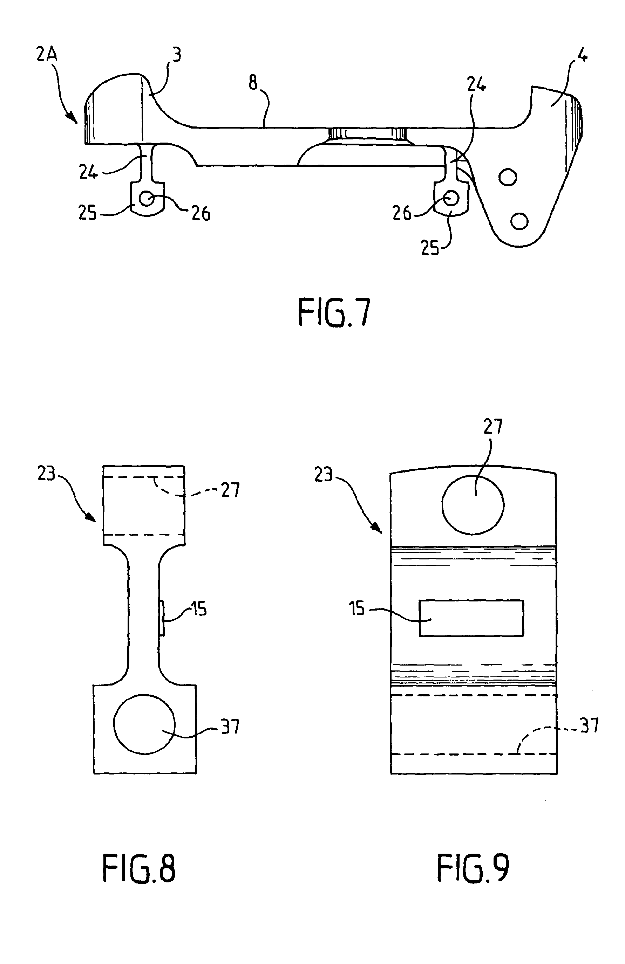

Afin de permettre le pivotement de l'organe de liaison dans cette articulation, l'extrémité supérieure de l'organe de liaison présente avantageusement, vu de côté, une surface incurvée vers le haut comme cela est illustré à la figure 9.

Selon une autre caractéristique de l'invention, la partie inférieure 2B du corps de pédale 2 a la forme d'une fourche 29 dont les branches 30, 31 sont dirigées vers l'avant et portent des jauges de déformation 19, 20, 21, 22 permettant de mesurer la flexion des branches 30, 31 dans un plan vertical.

Chacune des branches 30, 31 de la fourche 29 comporte avantageusement dans une partie médiane une fente horizontale 32 s'étendant dans l'axe longitudinal de la branche et limitée à chaque extrémité par une lumière circulaire 33, 34 permettant de déformer la branche selon une ligne brisée. Les jauges de déformation 19 à 22 sont disposées verticalement directement au-dessus des lumières respectives 33, 34.

Afin de désaccoupler les efforts transversaux des organes de liaison 23 vers le bas, ceux-ci sont reliés à la partie inférieure 2B du corps de pédale 2 par des deuxièmes articulations. Dans ce but, la partie inférieure 2B comporte des évidements 25 dirigés vers le haut et recevant l'extrémité inférieure d'un organe de liaison correspondant 23.

Chaque évidement a la forme d'un rectangle et constitue dans l'exemple illustré un trou traversant pourvu de deux alésages opposés 36 aligné sur un alésage 37 effectué dans l'extrémité inférieure de l'organe de liaison 23 pour recevoir un axe de pivotement longitudinal 38 s'étendant à travers les trois alésages 36, 37 de manière à former cette deuxième articulation.

Deux des évidements 25 sont disposés à proximité de l'extrémité libre d'une branche respective 30, 31 de la fourche 29, alors que le troisième évidement est effectué dans un appendice central 39 à l'arrière de la partie inférieure 2B du corps de pédale 2.

La pédale selon l'invention comporte avantageusement des moyens de mesure d'angle secondaires sous forme d'un codeur angulaire 40 associé à l'axe de pédale 10 et entièrement intégré dans le corps de pédale 2, entre la partie supérieure 2A et la partie inférieure 2B de celui-ci. Ce codeur angulaire permet, en combinaison avec les moyens de mesure de l'angle de rotation de la manivelle de pédalier 11, de déterminer à chaque instant l'angle d'inclinaison de la pédale à l'horizontale.

Toutes les jauges de déformation 12 à 22, ainsi que les moyens de mesure de l'angle de rotation de la manivelle de pédalier 11 et les moyens de mesure d'angle secondaires, sont reliés à une unité de numérisation et de mémorisation 40 qui selon l'invention est intégrée dans le corps de pédale 2.

Cette unité de numérisation et de mémorisation 40 est très schématiquement matérialisée par un boîtier électronique disposé dans l'interstice 2C entre la partie supérieure 2A et la partie inférieure 2B du corps de pédale 2. Ce boîtier électronique 40 comporte un logement 41 ouvert vers l'extérieur pour recevoir un support de mémoire amovible 42 sous forme d'une carte de mémoire.

Le corps de pédale 2 est fermé par le dessous par un couvercle 43 adapté à la forme du corps de pédale 2 et comportant sur sa face frontale une ouverture rectangulaire 44 permettant l'introduction de la carte de mémoire dans le logement 42 du boîtier 41.

De cette manière, l'ensemble des moyens de mesure des contraintes, de mesure de l'angle de rotation, ainsi que l'unité de numérisation et de mémorisation, sont embarqués sur le cycle afin de permettre acquisition et la sauvegarde des données, sans aucune liaison filaire vers l'extérieur, ce qui permet la mesure complète de la force appliquée contre la pédale en condition d'utilisation normale et sans fil perturbateur du mouvement.

La carte de mémoire amovible 42 peut après l'utilisation du cycle pourvu de la pédale selon l'invention sur le terrain dans des conditions réelles être enlevée pour être connectée à une unité de traitement de données et de visualisation (non représentée) apte à montrer les résultats de l'essai. Toutes les données sont transférées de la carte de mémoire 43 vers cette unité de traitement de données et de visualisation, suite à quoi les données sont effacées de la carte de mémoire qui ensuite peut être utilisée de nouveau.

Ainsi est obtenue une pédale dynamométrique autonome qui permet d'effectuer sur le terrain toutes les mesures souhaitables des trois composantes des forces appliquées sur la pédale et grâce à la séparation complète des mesures des efforts longitudinaux, transversaux et verticaux, on n'a plus besoin de matrice de calibrage pour calculer les valeurs et on obtient de cette manière des valeurs très exactes et extrêmement fiables.

Même si un seul mode de réalisation vient d'être décrit en détail, l'homme du métier peut, à partir de l'enseignement donné ci-dessus, concevoir d'autres variantes sans pour autant sortir du cadre de l'invention. Il serait, entre autres, possible de faire en sorte que les pattes verticales 24 portent des jauges de déformation s'étendant dans le sens longitudinal du corps de pédale afin de mesurer les efforts transversaux, alors que les organes de liaison verticaux 23 porteraient dans ce cas des jauges de déformation s'étendant dans le sens transversal du corps de pédale afin de mesurer les efforts longitudinaux.

Bien entendu, il est préférable d'utiliser une paire de pédales selon l'invention afin de permettre des mesures simultanées sur les forces exercées par les deux membres inférieurs du cycliste. De cette manière, il est possible de constater les dissymétries qui peuvent exister entre les deux membres inférieurs.

## Revendications

1. Pédale de cycle dynamométrique pour la mesure des efforts appliqués sur la pédale (1) lors du pédalage, comportant un corps de pédale (2) pourvu de moyens de fixation (3, 4) d'une chaussure de cycliste contre ledit corps de pédale (2), et d'un logement (9) recevant un axe de pédale (10) apte à être relié à une manivelle de pédalier (11), plusieurs jauges de déformation (12 à 22) étant disposées de part et d'autre de l'axe de pédale (10) et comprennent des jauges de déformation (12 à 14) pour mesurer les efforts longitudinaux, ainsi que des jauges (19 à 22) pour mesurer la flexion dans un plan vertical pour transformer les efforts reçus en signaux électriques à transmettre à une unité de numérisation des signaux et de mémorisation de données (41), la pédale étant en outre associée à des moyens de mesure de l'angle de rotation de ladite manivelle de pédalier (11), le corps de pédale (2) étant composé d'une partie supérieure (2A) comportant lesdits moyens de fixation (3, 4) et d'une partie inférieure (2B) comportant ledit logement (9), lesdites parties supérieure et inférieure (2A, 2B) du corps de pédale (2) étant séparées l'une de l'autre par un interstice (2C) tout en étant reliées l'une à l'autre par des organes de liaison verticaux (23), **caractérisée par le fait qu'**elle comporte en outre des jauges de déformation (15 à 17) aptes à mesurer les efforts transversaux et disposées dans ledit interstice (2C) en s'étendant dans le sens longitudinal du corps de pédale (2).

2. Pédale dynamométrique selon la revendication 1, **caractérisée par le fait que** lesdites jauges de déformation (15 à 17) aptes à mesurer les efforts transversaux sont portées par lesdits organes de liaison verticaux (23).

3. Pédale dynamométrique selon la revendication 1 ou 2, **caractérisée par le fait que** ladite partie supérieure (2A) du corps de pédale (2) est sur sa face inférieure pourvue de pattes verticales (24) reliées auxdits organes de liaison (23) et portant lesdites jauges de déformation (12 à 14) s'étendant dans le sens transversal du corps de pédale (2) afin de mesurer les efforts longitudinaux.

4. Pédale dynamométrique selon la revendication 3, **caractérisée par le fait que** lesdits organes de liaison (23) sont au nombre de trois et que deux des organes de liaison (23) sont disposés latéralement à l'avant de ladite partie supérieure (2A) du corps de pédale (2) et que le troisième organe de liaison (23) est disposé centralement à l'arrière de ladite partie supérieure (2A).

5. Pédale dynamométrique selon la revendication 4, **caractérisée par le fait que** ledit troisième organe de liaison (23) porte en outre une jauge de déformation (18) permettant de mesurer la compression.

6. Pédale dynamométrique selon l'une quelconque des revendications 3 à 5, **caractérisée par le fait que** lesdites pattes (24) sont reliées auxdits organes de liaison (23) par des premières articulations (25 à 28) désaccouplant les efforts longitudinaux de ladite partie supérieure (2A) du corps de pédale (2) vers le bas.

7. Pédale dynamométrique selon l'une quelconque des revendications précédentes, **caractérisée par le fait que** ladite partie inférieure (2B) du corps de pédale (2) a la forme d'une fourche (29) dont les branches (30, 31) sont dirigées vers l'avant et portent lesdites jauges de déformation (19 à 22) permettant de mesurer la flexion desdites branches (30, 31) dans un plan vertical.

8. Pédale dynamométrique selon la revendication 7, **caractérisée par le fait que** chacune desdites branches (30, 31) de ladite fourche (29) comporte dans une partie médiane une fente (32) s'étendant selon l'axe longitudinal de ladite branche (30, 31) et limitée à chaque extrémité par une lumière circulaire (33, 34) permettant de déformer la branche (30, 31) selon une ligne brisée.

9. Pédale dynamométrique selon l'une quelconque des revendications précédentes, **caractérisée par le fait que** lesdits organes de liaison (23) sont reliés à ladite partie inférieure (2B) du corps de pédale (2) à l'aide de deuxièmes articulations (35 à 38) désaccouplant les efforts transversaux desdits organes de liaison (23) vers le bas.

10. Pédale dynamométrique selon l'une quelconque des revendications précédentes, **caractérisée par le fait que** lesdits organes de liaison (23) sont des plaquettes allongées amincies dans leur partie centrale et s'étendant dans des plans longitudinaux de la pédale (1).

11. Pédale dynamométrique selon l'une des revendications 6 à 10, **caractérisée par le fait que** l'extrémité libre desdites pattes verticales (24) a la forme d'une chape (25) apte à recevoir l'extrémité supérieure de l'organe de liaison correspondant (23), et en ce que les branches de ladite chape comportent deux alésages opposés (26) alignés sur un alésage (27) effectué dans l'extrémité supérieure dudit organe de liaison (23) pour recevoir un axe de pivotement transversal (28) s'étendant à travers les trois alésages (26, 27) de manière à former ladite première articulation.

12. Pédale dynamométrique selon la revendication 11, **caractérisée par le fait que** l'extrémité supérieure de chaque organe de liaison (23) présente une surface incurvée vers le haut.

13. Pédale dynamométrique selon l'une des revendications 9 à 12, **caractérisée par le fait que** ladite partie inférieure (2B) du corps de pédale (2) comporte des évidements (35) dirigés vers le haut et recevant l'extrémité inférieure d'un organe de liaison correspondant (23), chaque évidement (35) étant pourvu de deux alésages opposés (36) alignés sur un alésage (37) effectué dans ladite extrémité inférieure dudit organe de liaison (23) pour recevoir un axe de pivotement longitudinal (38) s'étendant à travers les trois alésages (36, 37) de manière à former ladite deuxième articulation.

14. Pédale dynamométrique selon l'une quelconque des revendications précédentes, **caractérisée par le fait que** ladite unité de numérisation et de mémorisation (41) est intégrée dans le corps de pédale (2).

15. Pédale dynamométrique selon la revendication 14, **caractérisée par le fait que** ladite unité de numérisation et de mémorisation (41) comporte un boîtier électronique associé à un support de mémoire amovible (43).

16. Pédale dynamométrique selon l'une quelconque des revendications précédentes, **caractérisée par le fait que** la pédale (1) comporte des moyens de mesure d'angle secondaires (40) aptes à mesurer l'angle de l'axe de pédale (10) par rapport au corps de pédale (2) et à générer des signaux électriques à transmettre à ladite unité de numérisation des signaux et de mémorisation de données.

17. Pédale dynamométrique selon la revendication 16, **caractérisée par le fait que** lesdits moyens de mesure d'angle secondaires (40) sont sous forme d'un codeur angulaire intégré dans le corps de pédale (2).
